# EUROPEAN PATENT SPECIFICATION

(11) **EP 1 715 878 B1**
(45) Date of publication and mention of the grant of the patent: **11.03.2009**
(21) Application number: 04806442.2
(22) Date of filing: 27.12.2004
(51) Int. Cl.: A61K 36/488, A61K 31/70, A61P 3/10

(54) **USE OF THE LUPINOSIDE PA4 OR A BUTANOL FRACTION OF AN EXTRACT OF PUERARIA TUBEROSA FOR THE TREATMENT OF DIABETES TYPE 2**
VERWENDUNG DES LUPINOSIDS PA4 ODER EINER BUTANOL-FRAKTION EINES PUERARIA TUBEROSA-EXTRAKTS ZUR BEHANDLUNG VON TYP-2 DIABETES
UTILISATION DU LUPINOSIDE PA4 OU D'UN FRACTION BUTANOLIQUE D'UN EXTRAIT DE PUERARIA TUBEROSA POUR LE TRAITEMENT DU DIABÈTE DU TYPE 2

(30) Priority: 09.01.2004 US 535332 P
(43) Date of publication of application: 02.11.2006
(73) Proprietor: Council of Scientific and Industrial Research, New Delhi 110 001, Maharashtra (IN)
(72) Inventor: BHATTACHARYA, Samir, Kolkata 700 032 (IN); DEY, Debleena, Indian Instit. of Chemical Biol., Kolkata 700 032 (IN); MANDAL, Swapan Kumar, Kolkata 700 032 (IN); MUKHERJEE, Mohua, Indian Inst. of Chemical Biol., Kolkata 700 032 (IN); PAL, Bikash Chandra, Kolkata 700 032 (IN); BISWAS, Tanushree, Kolkata 700 032 (IN); DATTA, Malabika, Kolkata 700 032 (IN); ROY, Sib Sankar, Kolkata 700 032 (IN); BANDYOPADHYAY, Arun, Kolkata 700 032 (IN); BANDOPADHYAY, Santu, Kolkata 700 032 (IN); KONAR, Aditya, Kolkata 700 032 (IN); GIRI, Bir Bhanu, Purulia, West Bengal 700 032 (IN)
(74) Representative: Behnisch, Werner
(86) International application number: PCT/IB2004/004273
(87) International publication number: WO 2005/074958

(56) References cited:
- WO-A-03/017784
- US-A1- 2003 185 913
- NAHAR PHARMACEUTICALS: "DIABEEN" INTERNET ARTICLE, [Online] 13 December 2003 (2003-12-13), XP002329344 Retrieved from the Internet: URL:http://naharpharma.com/patent_formulat ions.htm> [retrieved on 2005-05-17]
- NADAN HERBAL PHARMACY: "Pueraria tuberosa" INTERNET ARTICLE, [Online] 17 February 2003 (2003-02-17), XP002329345 Retrieved from the Internet: URL:http://www.nadanherbcomp.com.au/herb-p .html> [retrieved on 2005-05-18]
- SHUKLA, S. ET AL.: "Butanolic Extract of Pueraria tuberosa DC.: Physiological Response in the Genital Tract of Cyclic Female Rats" PHYTOTHERAPY RESEARCH, vol. 3, no. 1, 1989, pages 5-10, XP008047503
- KINJO, J. ET AL.: "Study of Structure-Hepatoprotective Relationships of Oleanene-Type Triterpenoidal Glucuronides Obtained from Several Fabaceous Plants on Rat Primary Hepatocyte Cultures" BIOLOGICAL AND PHARMACEUTICAL BULLETIN, vol. 22, no. 2, February 1999 (1999-02), pages 203-206, XP008047269
- KINJO, J. ET AL.: "Five New Triterpene Glycosides from Russell Lupine" CHEMICAL AND PHARMACEUTICAL BULLETIN, vol. 42, no. 9, September 1994 (1994-09), pages 1874-1878, XP008047299 cited in the application
- GUPTA, D.N. ET AL.: "Postcoital contraceptive efficacy and hormonal profile of Pueraria tuberosa" INDIAN DRUGS, vol. 27, no. 7, 1990, pages 372-374, XP008051624

## Description

### Field of the invention

The present invention relates to the use of an effective amount of active n-butanol fraction of Pureria tuberosa and active molecule Lupinoside PA4 (LPA₄), useful in preventing and/or treating diabetes type 2; and lastly, a pharmaceutical composition containing n-butanol fraction of an extract of Pureria tuberosa .

### Background and Prior art references of the Present Invention

Type 2 insulin-resistant diabetes mellitus, an insidious disease, accounts for more than 95 % of diabetic cases. This heterogeneous disorder is increasing in epidemic proportions, its world wide frequency is expected to grow more than six percent per anum^{1,2}. The disease is primarily expressed in the form of hyperglycemia due to defects in glucose disposal into skeletal muscle, fat and liver as they become less responsive or resistant to insulin^{3,4}. Large number of evidences has been accumulated that hold free fatty acids (FFAs) responsible for insulin inaction. Elevated FFAs in circulation is associated with impaired insulin function and is commonly linked with obesity and type 2 diabetes⁵⁻⁷. Rising of plasma FFA concentrations through lipid infusion causes insulin resistance in rat and human skeletal muscle⁷⁻⁹. Incubation of isolated muscle strips or cultured muscle cells with FFAs or lipoprotein lipase expression in skeletal muscle reduces insulin-mediated glucose uptake⁶⁻¹². These reports suggest that greater deposition of lipid in insulin sensitive tissues promotes insulin inaction and resistance. FFA induced impairment of insulin activity appears to be associated with insulin signaling defects. Lowering of glucose transport by FFA is linked to inhibition of insulin-stimulated IRS-1 phosphorylation and IRS-1 associated phosphatidylinositol-3-phosphate kinase (PI3K) activation¹³⁻¹⁵. Thiazolidinedione (TZD) treatment reduces circulating FFAs that oppose insulin actions in target tissues improving insulin activity¹⁶⁻¹⁸. By inducing the glycerol kinase gene expression in adipocytes through the activation of PPARγ, TZD augments glycerol incorporation into triglyceride thus reduces FFA secretion from the adipocyte and that helps insulin sensitization¹⁹ The document US 2003/0185913 A1 (Pushpangadan, P. & Prakash, D., 2 October 2003) discloses pharmaceutical compositions comprising aqueous alcoholic extracts of *Pueraria tuberosa* and the use of these compositions for the treatment of diabetes type 2.

### Objects of the present invention

The main object of the present invention is to provide a new use for lupinoside PA4.

Still another object of the present invention is to develop a pharmaceutical composition for preventing and/or treating diabetes type 2.

### Summary of the present invention

The present invention relates to the use of a pharmaceutically effective amount of butanol fraction of extract of plant Pueraria tuberosa or Lupinoside PA4, optionally along with pharmaceutically available additive(s) for the manufacture of a medicament for preventing and/or treating diabetes type 2 in a subject in need thereof.

### Detailed description of the Present Invention

In one embodiment of the present invention the subject is an animal.

In another embodiment of the present invention the subject is a human being.

In still another embodiment of the present invention the fraction is administered at the concentration ranging between 1 to 40 mg /kg body weight.

In still another embodiment of the present invention the invention relates to a method as claimed in claim 1, wherein the Lupinoside is administered at the concentration ranging between 1 to 40 mg /kg body weight.

In still another embodiment of the present invention the invention relates to a method as claimed in claim 1, wherein the administration route is selected from a group comprising orally, intravenously, intramuscularly, and subcutaneously.

In still another embodiment of the present invention wherein the invention relates to a pharmaceutical composition useful in preventing and/or treating diabetes type 2, said composition comprising butanol fraction of the extract of plant *Pueraria tuberosa* containing Lupinoside PA4 (LPA₄), and additive(s).

In still another embodiment of the present invention the additive is selected from a group comprising nutrients such as proteins, carbohydrates, sugars, talc, magnesium stearate, cellulose, calcium carbonate, starch, gelatin paste, pharmaceutically acceptable carrier, excipients, diluent and, solvent.

In still another embodiment of the present invention the extract is obtained from root of the plant.

In still another embodiment of the present invention the fraction is of concentration ranging between 1 to 40 mg /kg body weight.

In still another embodiment of the present invention the Lupinoside is of concentration ranging between 1 to 40 mg /kg body weight.

In still another embodiment of the present invention the composition is in a form selected from a group comprising capsule, syrup, concentrate, powder, and granules.

In still another embodiment of the present invention the extract is an aqueous extract.

It has been discovered that the mode of action comprises augmenting Glut4 phosphorylation and Glut4 translocation to a target cell membrane to enhance insulin signal in a signal transduction pathway in a subject in need thereof, by administering pharmaceutically effective amount of butanol fraction of the extract of *Pueraria tuberosa* or Lupinoside PA4 (LPA₄), optionally along with additive(s) to the subject.

An increase in glucose uptake by the cells is shown.

The use is non-toxic to the cells.

The translocation is from cytosol to membrane of the insulin response cells.

The Lupinoside PA4 (LPA₄) prevents palmitate induced defects on insulin signaling.

The Lupinoside PA4 (LPA₄) allows insulin so stimulate IR-beta and Akt phosphorylation.

In still another embodiment of the present invention, the active

butanol fraction and active molecule Lupinoside PA4 (LPA₄), useful in preventing and/or trating diabetes type 2, is obtained by a process comprising tte steps of
- cutting the plant parts into small parts,
- extracting the cut parts with methanol and water,
- partitioning the methanol and water extract between ethyl acetate and water,
- extracting the aqueous layer further with n-butanol to obtain butanol fraction, and
- subjecting the n-butanol fraction to chromatography with water and methanol as eluent to obtain Lupinoside PA₄ (LPA₄).

In still another embodiment of the present invention the plant part is root.

In still another embodiment of the present invention the solvent is selected from a group comprising methanol, and water.

In still another embodiment of the present invention the water and methanol are in the ratio of about 1:1.

In still another embodiment of the present invention the chromatogaphy is column chromatography.

The decrease in insulin sensitivity to target tissues or insulin resistance leads to diabetes type 2, a disease now reaching to epidemic proportions in industrialized societies. It is still unclear how insulin loses its sensitivity. A large number of evidences made free fatty acids (FFAs) responsible for insulin resistance. We have demonstrated that palmitate, one of the FFAs, interfered with insulin binding to 210 kDa receptor protein from 3T3L1 adipocyte cell membrane. Palmitate did not alter affinity of insulin binding as Ka remains unchanged, but it drastically reduced insulin occupation of receptor from Bmax 7.3 pM (insulin) to 3.46 pM (insulin plus palmitate). Inhibition of interaction of insulin with insulin receptor (IR) by palmitate coincided with the reduction of IRβ tyrosine phosphorylation, a critical target cell response followed by insulin-IR complex. We then examined insulin stimulated downstream signals, which are consequently phosphorylated following IRβ tyrosine phosphorylation. A 24h incubation of 3T3L1 cells with palmitate affected about two-fold decrease of insulin-augmented IRS 1, PI3 Kinase and Akt phosphorylation and completely blocked insulin-induced Glut4 translocation. All these indicate downing of insulin signals by palmitate and that causes insulin inaction. Lupinoside PA4 (LPA₄), isolated from a plant root, prevented palmitate-induced defects on insulin signaling. LPA₄ co-incubation with palmitate allowed insulin to stimulate IRβ and Akt phosphorylation, and insulin-induced Glut4 translocation. Hence, LPA₄ shows a promise for its use as a therapeutic agent in insulin resistance and diabetes type 2.

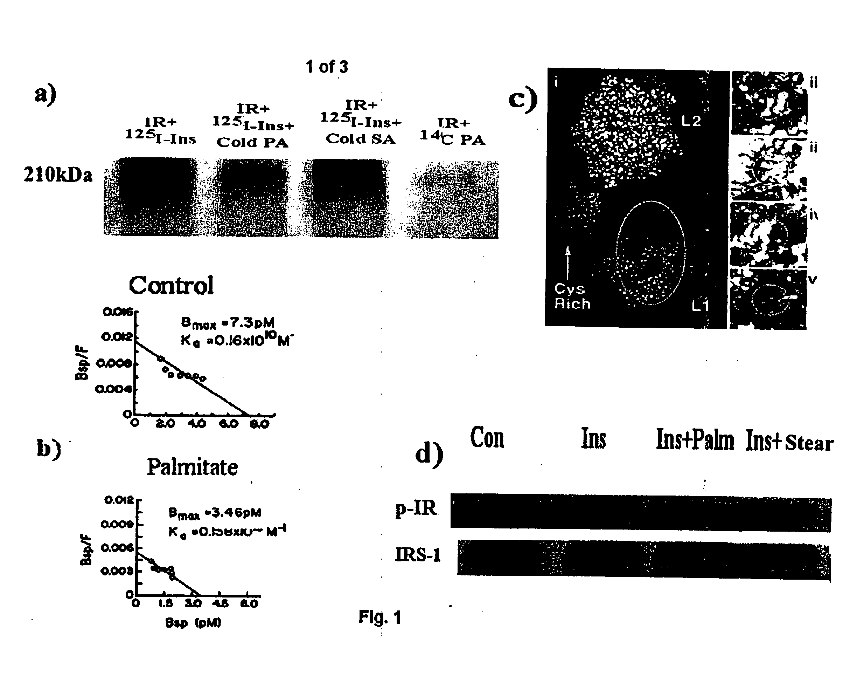

These reports drive our attention towards FFAs as the principal compound causing insulin resistance and diabetes type 2. Earlier reports indicated that among the FFAs, palmitate is the most potent inhibitor of insulin activity^{12,20-23}, but the question confronting us is how it imposes such defects. A 24h pre-treatment of 3T3L1 adipocytes with palmitate, myristate, butyrate, caprylate, stearate, laureate and linoleate separately followed by 30min incubation with insulin and then determination of ³H-2deoxyglucose (2-DOG) uptake showed that palmitate could be singled out as the most potent inhibitor (data not shown). This led us to search for the underlying mechanisms responsible for palmitate-induced inhibition of insulin stimulated glucose uptake. Palmitate incubated adipocytes were lysed, membranes were isolated, solubilised, subjected to non-denaturing SDS-PAGE then subjected to autoradiography. ¹²⁵I-insulin bound protein could be located at 210 kDa region of the gel that corroborates earlier reports of detergent-solubilised insulin receptor under non-denaturing conditions with 3T3-L1 adipocytes²⁴⁻²⁶. Figla shows that palmitate effectively reduced insulin binding to the receptor. Palmitate binding or palmitoylation of IR, as reported with β-adrenergic receptor²⁷⁻²⁹, did not alter the affinity but effected two-fold decrease in receptor occupation, Bₘₐₓ was reduced from 7.3 to 3.46 pM (Fig.1b). Since ¹²⁵I-insulin binding to the receptor could not be reduced by stearate or myristate (data not shown), we presume this to be a specific palmitate effect. Interestingly, radiolabelled palmitate binds to similar molecular size protein (1a) suggesting palmitoylation of IR. With the help of a model, the logic behind this postulation is explained in Fig. 1c. In order to identify the probable site for palmitoylation, 3-D structure of the first three domains of rat insulin receptor was homology modeled based on the recently determined crystal structure of the type-1 insulin-like growth factor receptor³⁰. Alanine scanning mutagenesis results strongly suggest that this area on the insulin receptor (within the cyan ring in Fig. 1c) is the most probable site for insulin binding³¹. The lack of consensus amino acid sequence²⁷ implies that palmitoylation preference is determined by the 3-D structure and it would be favored by a positively charged and/or neutral surface. The calculation of surface electrostatic potential of the modeled structure, solvent accessibility and proximity measurement of the cysteine residues have helped us to identify two potential pairs of Cys residues (Cys-8 & Cys-26 and Cys-266 & Cys-274) for palmitoylation out of 16 such pairs in the L1-Cys rich-L2 regions of the IR. Since mere association of palmitate with IR does not imply biological relevance, we studied palmitate interference on insulin stimulated signals to demonstrate the functional significance correlated with its binding. We first observed phosphorylation of insulin receptor β (IRβ) by incubating 3T3L1 cells without or with palmitate for 24h, followed by insulin incubation. Insulin-stimulated phosphorylation of IRβ was dramatically reduced by palmitate while stearate did not exhibit such inhibition (Fig 1d). These results suggest palmitate association or palmitoylation of the receptor has physiological relevance since IRβ phosphorylation is a critical response from insulin target cell.

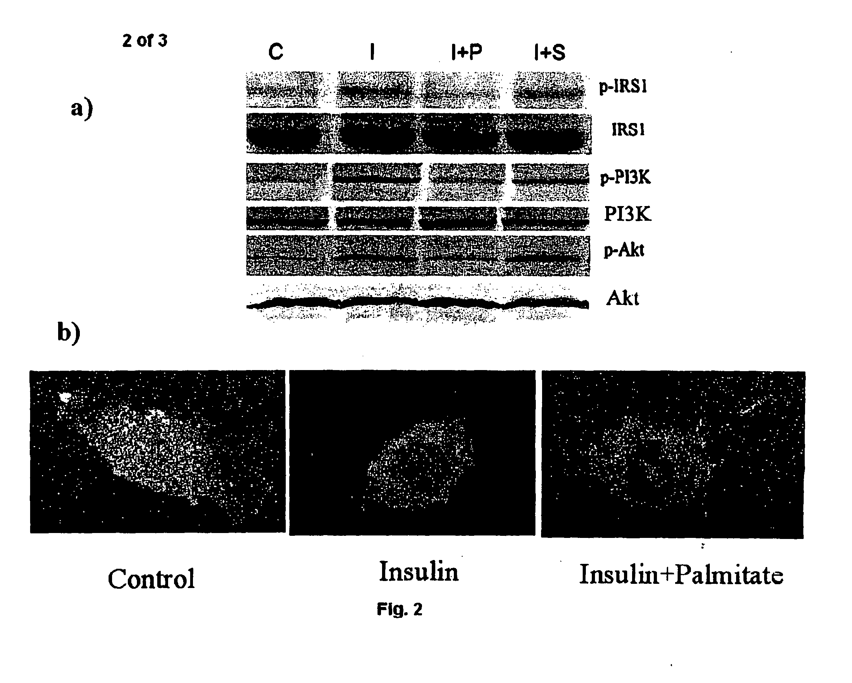

To have further evidence, we examined insulin augmented downstream signals, which get consequently phosphorylated following receptor tyrosine kinase phosphorylation. Using phospho-specific antibodies, we found that insulin stimulation of IRS-1 phosphorylation and PI3K activity was significantly inhibited by palmitate. Palmitate also reduced insulin stimulation of other downstream molecules namely, Akt activation. However, incubation with stearate showed no such inhibitory effect (Fig.2a). Decrease of insulin stimulation of IRS associated phosphorylation and IRS1 associated PI3 K activity by FFA has been reported earlier ¹³, ¹⁵, ²⁰, here we show that palmitate alone can produce such defects. Another interesting trend observed in our study is the closeness of range of palmitate-induced inhibition. Densitometric analysis of Western blots indicates a two-fold decrease of insulin-stimulated phosphorylation of IRβ, PI3 K and Akt phosphorylation (data not shown). Neither insulin nor palmitate affected any alteration of protein profiles of IRβ, PI3 K and Akt (Fig. 2a). Our findings give an impression that palmitate disruption of insulin signals probably originates at IR level and the wave of inhibition then flows through the down stream signaling molecules. This restricts the recruitment of PI3 kinase resulting inhibition of PIP₃ association with Akt, which is expected to have adverse effect on Glut4 trafficking. Finally, a support in this direction was obtained with Glut4 translocation, which is essential for glucose entry into the cell. Insulin induced the translocation of GFP-Glut4 from the cytosol to the adipocyte membrane was totally inhibited by palmitate(Fig 2b).

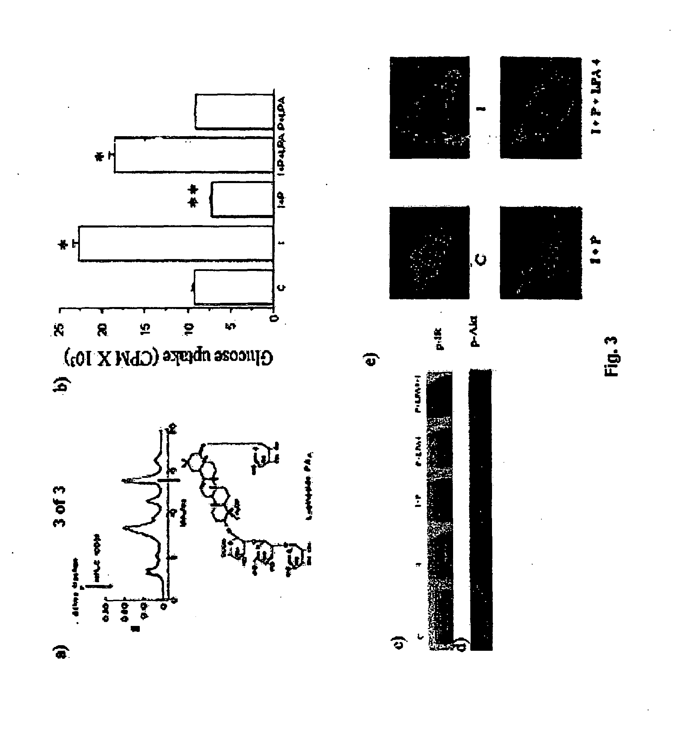

In the process of searching for anti-diabetic activity of medicinal plants of India, methanol-water (1:1) extract from *Pueraria tuberosa* root was found to improve palmitate impairment of insulin activity in terms of ³H-2DOG uptake by 3T3L1 cells. Using Diaion HP-20 chromatography, we obtained five fractions (A-E) of which fraction E showed required activity. Fractionation of E through Sephadex LH 20 chromatography yielded 3 fractions (F-H) where F showed improvement of palmitate induced damage. Fraction F was subsequently purified by HPLC to a single molecule, which was identified as Lupinoside PA₄³² by 2D NMR and mass spectrometry (Fig.3a). LPA₄ protective property on the palmitate-induced impairment of insulin signaling molecules was then examined on 3T3L1 adipocytes. Fig 3b demonstrates palmitate-induced reduction of insulin augmented ³H-2-DOG uptake by adipocyte could be prevented by LPA₄. Attenuating effect of palmitate on insulin-stimulated IRβ tyrosine and Akt phosphorylation was waived by LPA₄ (Fig. 3 c).

Akt is known to be a very important downstream signal, which activates Glut4, a process essential for its translocation to the plasma membrane in insulin target cells^{33,34}. In response to insulin, Akt 2 is recruited to Glut4 containing vesicles and phosphorylates the component proteins³⁵. Hence LPA₄ influence in waiving palmitate-induced inhibition of insulin stimulated Akt phosphorylation is meaningful, as damage in this pathway cause insulin resistance. This is further supported by our observation on palmitate inhibited insulin stimulation of Glut4 translocation. LPA₄ co-incubation with palmitate clearly permitted insulin stimulation of GFP-Glut4 translocation from cytoplasm to membrane (Fig. 3d). Glut4 is a vital transporter of glucose in insulin responsive cells, which constitutively recycles via the cell surface. Insulin actively sequesters Glut4 at an intracellular location that increase the rate of Glut4 trafficking to the membrane³². Although cellular mechanisms related to Glut4 trafficking events remain largely enigmatic but association of Glut4 translocation defects is involved in insulin resistance³⁴. Our results with the determination of GFP-Glut4 translocation demonstrate complete block of insulin-stimulated Glut4 translocation by palmitate and that is totally withdrawn by LPA₄. These results are of particular interest in insulin resistance or diabetes type 2 for the following reasons: (i) Large number of studies implicates FFAs, particularly palmitate, in the development of insulin resistance ²⁰⁻²³ ; (ii) Palmitate is the most abundant FFAs found in circulation and skeletal muscle cells^{10,12} ; (iii) one of the most prevalent acyl chains in diglyceride fraction of lipid extracts is palmitate³⁶; (iv) consumption of palmitate decreases insulin sensitivity ^{37,38} and (v) insulin resistant muscles exhibit greater rates of palmitate uptake ³⁹. All these reports draw attention towards palmitate to be the major candidate among FFAs causing insulin resistance. However, a few studies indicate palmitate induced defects in insulin action is mediated by ceramide¹². There may be number of pathways operative in insulin inaction, ceramide may be one of them and what we have observed is yet another, where palmitate is directly involved in causing insulin inaction. LPA₄ rescues insulin inactivation effected by palmitate. Since LPA₄ prevents insulin-signaling defects due to palmitate at all important steps including Glut4 translocation from cytoplasm to membrane, this lupinoside has encouraging possibilities as a therapeutic agent for insulin resistance and diabetes type 2.

### Detailed description of the accompanying drawings

- **Figure 1**.: (a) Palmitate induced inhibition of insulin binding to receptor and receptor tyrosine kinase phosphorylation. Autoradiograph of radiolabeled insulin and palmitate binding to solubilized insulin receptor preparations. 3T3 L1 adipocytes were incubated for 24 h without or with palmitate and receptor preparations were solubilized with 0.1% TritonX-100. 25 µg of protein was incubated overnight at 4°C with 2 ng ¹²⁵I-insulin (Specific activity 30.55µLCi/µg of protein). After termination of the incubation, it was pelleted by ultra centrifugation at 10⁵ g for 1 h. ¹²⁵I-insulin was bound to the solubilized receptor preparation. Insulin receptor preparation and ¹²⁵I-insulin was incubated in an identical manner in presence of cold palmitate (PA) or stearate (SA). 25 ug protein of solubilized receptor preparation was incubated overnight with [1-¹⁴C]-palmitate at 4°C and autoradiographed (¹⁴C-PA).
(b) Determination of the binding affinity and receptor occupation by Scatchard analysis. Bₘₐₓ and Kₐ were calculated to be 7.3pM and 0.16x10¹⁰M⁻¹ respectively for insulin binding to solubilized receptor. But in the presence of palmitate, Kₐ of insulin binding remained almost unchanged i.e. 0.158x10¹⁰M⁻¹ but the Bₘₐₓ was reduced to 3.46pM.
(c) Panel i presents the homology model of the three domains (L1-Cys rich-L2) of the insulin receptor. The region on the L1 domain marked by the cyan band is the most probable insulin-binding site. Residues which on mutation to alanine reduced the binding constant by about 300 fold are coloured red; those which on mutation lowered the binding between 10 to 100 fold are coloured pink and the rest which reduced binding by 3 to 9 fold are coloured yellow. Cysteine residues are coloured green of which Cys-8 & Cys-26 (marked as ii within the red circle) and Cys-266 & Cys-274 (marked as iii within the red circle) are the most probable palmitoylation sites. Panels (ii) and (iii) show the electrostatic potential of the surroundings of the two probable pairs of cysteines, Cys-8 & Cys-26 and Cys-266 & Cys-274 which were selected as palmitoylation site for their dominant positive (blue) and neutral (white) electrostatic potential environment. Panel (iv) shows the electrostatic potential environment panel of Cys-192 & Cys-201, although it has right (positive and neutral) environment it was not selected as potential site as is fully buried under the surface. Panel (v) shows the electrostatic potential around Cys-126 & Cys-155 pair, which represents those discarded sites, which are predominantly negatively charged (red).
(d) Control and FFA treated 3T3L1 adipocytes were lysed by sonication in lysis buffer and centrifuged for 10min at 10,000 g. 200µg supernatant protein from control and treated cells was incubated overnight at 4°C with 2µg IRβ antibody. The antigen-antibody complex was pelleted with Protein A-agarose, pellets were washed thoroughly, resuspended and boiled in SDS-PAGE sample buffer and
   electrophoresed. Proteins from the gel were transferred to PVDF membrane and immunoblotted with anti-p-Tyr antibody. I-Insulin; P-palmitate; S-stearate.
- **Figure 2**.: (a) 3T3L1 adipocytes were incubated with palmitate and stearate as described in Fig. 1. On termination of incubation, cells were lysed by sonication in lysis buffer and centrifuged at 10,000 g for 10min. Supernatant protein (50µg each) was boiled for 5min in SDS-PAGE sample buffer and resolved on 12% gel SDS-PAGE followed by transfer to PVDF membranes and immunodetected with p-IRS-1 (1:1000), p-PI3 kinase p 85 α (1:1000) and p-Akt 1/2 (1:1000) antibodies using alkaline phosphatase linked secondary antibodies. Anti-IRS 1, PI-3K and Akt 1/2 antibodies were used to detect the protein profiles due to treatments.
b) Effect of palmitate on insulin induced Glut4 translocation in 3T3L1 adipocytes. 3T3L1 cells plated on coverslips were transfected for 48 h with GFP-Glut 4 plasmid (2 µg) using lipofectamine reagents. On stabilization, the cells were incubated in the absence or presence of palmitate for 24 h and then with insulin for 30 min. GFP-Glut 4 transfected cells incubated in the absence of fatty acids and insulin served as control (Con). After termination of incubation, localization of GFP-Glut 4 was examined using laser scanning confocal microscope.
- **Figure 3**.: (a) Structure and purification of LPA₄. Using Diaion HP-20 chromatography, obtained five fractions (A-E) of which fraction E showed required activity. Fractionation of E through Sephadex LH 20 chromatography yielded 3 fractions (F-H) where F showed improvement of palmitate induced impaired insulin activity. Fraction F was subsequently purified by HPLC to a single molecule, which was identified as Lupinoside PA₄³² by 2D NMR and mass spectrometry
(b) Adipocytes were treated for 24h in the presence of palmitate or palmitate plus LPA₄ or LPA₄ followed by 30min insulin incubation. ³H-deoxyglucose was added to each incubation 5min prior to the termination of incubation. Cells were then washed thrice with ice-cold KRP buffer in the presence of 0.3mM phloretin to correct the glucose uptake data from simple diffusion and non-specific trapping of radioactivity. Cells were solubilized with 1% NP-40 and radioactivity was counted in a liquid scintillation counter.
(c) 3T3L1 adipocytes were incubated for 24 h in the absence or presence of palmitate or LPA₄ or palmitate plus LPA₄ and then with insulin 30 min. 50µg of cell lysate in each case was subjected to denaturing gel and transferred to PVDF membrane and immunoblotted with anti- p-Akt antibody. 50µg of cell lysate was immunoprecipitated with anti-IR β antibody and immunoblotted with anti-p-Tyr antibody.
(d) Glut 4 translocation of treated cells was determined in a similar manner as described under Fig. 2b.

### METHODS

**Cell Culture and treatments.** 3T3-L1 cell line was procured from the National Centre for Cell Science, Pune, India and was cultured at 37°C in 95%O₂/5% CO₂ in Dulbecco's Modified Eagle's Medium (DMEM) containing 25 mM glucose and 10% fetal calf serum. Confluent cells were treated, wherever mentioned, with 0.75mM free fatty acids (FFAs; palmitate and stearate) for 24h.

**Radiolabeled insulin binding to solubilized receptor preparations.** Control and FFA treated 3T3L1 adipocytes were first washed thrice with 0.02M Phosphate Buffer (pH-7.4) containing 0.14M NaCl and then resuspended in Lysis Buffer (20mM Tris-HCl, 40mM NaCl, 5mM EDTA, 5mM Iodoacetamide, pH-8.4) supplemented with protease inhibitors (1µg/ml aprotinin, 1µg/ml pepstatin, 1µg/ml leupeptin, 2mM phenyl methyl sulfonyl fluoride and 1µg/ml trypsin inhibitor). These cells were then freeze thawed thrice at -70°C and centrifuged at 10,000rpm for 15min at 4°C. The pellet collected was resuspended in Lysis Buffer, sonicated and again centrifuged at 10,000rpm for 15-20min at 4°C. The supernatant was dialysed overnight against 10mM Tris-HCl (pH-7.4) buffer and volume reduced by lyophilisation. The membrane preparation was then mixed with 0.1M lithium diiodosalicylate to a membrane protein concentration of approximately 5mg/ml and the mixture was homogenised in a motor driven glass-Teflon tissue homogenizer, it was centrifuged at 35,000g for 20min and supernatant was dialysed for 12hours against 20mM sodium bicarbonate, pH-9.4. To this solution 0.1% TritonX-100 (v/v) and 25% glycerol was added with constant stirring. The solution was lyophilised to reduce the volume and subjected to dialysis against 10mM Tris-HCl buffer (pH-8.4). Recombinant human insulin was radiolabelled with ¹²⁵I and ¹²⁵I-insulin was separated from free iodine by using Sephadex-G15 column equilibrated with 0.01M Phosphate Buffer (pH-7.2) containing 0.14M NaCl and 1% (w/v) BSA. Specific activity of ¹²⁵I-insulin was 30.55µCi/µg of protein.

Solubilized insulin receptor preparation from control and FFA treated 3T3L1 adipocytes (25µg for each incubation) was incubated overnight at 4°C with 2ng ¹²⁵I labeled recombinant human insulin in a final volume of 500µl of 0.02M Phosphate Buffer (pH-8.4) containing 0.15M NaCl and 0.25% BSA (PBS). On termination of incubation, it was pelleted by ultracentrifugation (Sorvall Ultra-80) at 10⁵g for 1hour. Pellet in each tube was dissolved in 1X sample buffer (63mM Tris HCl pH 6.8, 10% glycerol, 2% SDS, and 0.025% Bromophenol blue) and subjected to non-denaturing SDS-PAGE (4% stacking gel was layered on the top of a 6.5% resolving gel). The gel was dried, exposed to Kodak X-OMAT AR and autoradiographed (see **Fig1a**).

**Scatchard Analysis**. To determine the optimum binding conditions of ¹²⁵I-Insulin to the receptor protein, binding incubations were performed at different temperatures and time intervals with varied amount of solubilized receptor preparations. It was found that overnight incubation at pH 8.4 and a temperature of 4°C permitted maximum radiolabeled insulin binding. The receptor preparations (15µg protein) was incubated overnight at 4°C in a final volume of 500µl buffer (0.02M Phosphate Buffer, pH 8.4, containing 0.15M NaCl and 0.25% BSA) with varying concentrations of ¹²⁵I-Insulin (0.18-0.72 nmoles/L) in the absence (total binding) or presence of 10,000fold excess unlabeled insulin (non-specific binding). In another set of experiment receptor preparation was incubated simultaneously with ¹²⁵I -Insulin and unlabeled insulin except the presence of 0.08mM cf unlabeled palmitate. After the termination of the incubations, free and bound radioactivity was separated by the addition of 500µl of 0.5% chilled polyethylene glycol (PEG; MW 6000). The samples were mixed thoroughly by vortexing and kept under ice for 10min and followed by centrifugation at 20,000g in a refrigerated centrifuge for 15min. The supernatant was aspirated out and the pellet was washed three times with washing buffer (0.02M Phosphate Buffer containing 0.15M NaCl and 0.25% BSA). The radioactivity in the final pellet was measured in a ¹²⁵I-gamma counter. The specific binding was calculated by subtracting non-specific binding from total binding. Data were then analyzed by Scatchard analysis to determine the affinity and capacity of insulin receptor binding in the presence or absence of palmitic acid (see **Fig1b**).

**Molecular modeling**. The homology model of the insulin receptor was done on the x-ray structure (PDB1IGR.ENT, with amino acid identity of 59%) of IGF-1R²⁷ using InsightII 98.0 (Accelrys Inc., San Diego, CA, USA). Energy minimization and molecular dynamics were performed with the DISCOVER module of InsightII using cff91 forcefield on a Silicon Graphics^{R} OCTANE workstation. Energy minimizations were done with a convergence criterion of 0.001 kcal/mol, using a combination of steepest descent and conjugate gradient methods (100 steps each); these steps were repeated until satisfactory conformational parameters were obtained. Molecular dynamics simulations were carried out using a time step of 1 fempto second for 100 steps of equilibration and 1000 steps of dynamics. Distance constraints were applied to the other parts of the molecule while running minimization and dynamics for regularization of selected segments (see **Fig. 1c**).

**Immunoprecipitation.** 200 µg of control and FFA treated cell lysates (sonicated in ice for 10 min in lysis buffer [1% NP-40, 20 mM HEPES (pH 7.4), 2 mM EDTA, 100 mM NaF, 10 mM sodium pyrophosphate, 1 mM sodium orthovanadate, 1 µg/ml leupeptin, 1 µg/ml aprotinin, 1 µg/ml pepstatin and 1 mM PMSF] followed by) were incubated overnight at 4°C with 2 µg insulin receptor (IR) β antibody. 50 µl of Protein A-agarose was added to each tube and incubated at 4°C for 2 h. After centrifugation at 10,000 g for 2 min at 4°C, 500 µl of 0.1% CHAPS in PBS was added to the pellets, resuspended and centrifuged at 10,000 g at 4°C for 2 min. The pellets were washed thoroughly and subjected to SDS-PAGE followed by Western Blot using anti-phosphotyrosine antibody (anti-mouse; 1:1000) (see **Fig1d**).

**Electrophoresis and Immunoblotting.** Control and treated cell lysates (60 µg) where resolved on 10% SDS-PAGE and transferred to PVDF membranes (Millipore, Bedford, MA 01730) in transfer buffer (25 mM Tris, 193 mm glycine, 20 % methanol, pH 8.5) for 1.5 h at 4°C at 90 V. Membranes were blocked with 5% non-fat dried milk in TBST buffer (20mM Tris base, 137mM NaCl, 1mM HCl, 0.1% Tween 20) and incubated overnight with anti p-IRS (anti-goat; 1:1000), anti p-PI3K (anti-goat; 1:1000) and anti p-Akt (anti-rabbit; 1:2000). Immunoreactive bands were detected with alkaline phosphatase linked secondary antibodies (see **Fig 2a**).

**Transfection and Glut 4 translocation.** 3T3L1 Cells were plated on 60 mm plate containing coverslips and maintained in an air/CO₂ (19:1) atmosphere in DMEM supplemented with 10% (v/v) FBS and 100µg/ml penicillin/streptomycin. After 24 hrs, cells were washed with DMEM free from FBS and antibiotics. Plasmid DNA of GFP-Glut4 (2 µg) was used to transfect 2x10⁵ cells on each 60 mm plate with Lipofectamine reagent in accordance with the manufacturer's protocol (Life Technologies). After 48 h of transfection, cells were treated without or with 0.75mM palmitate for 24 h and then incubated for 30 min in the absence or presence of 100nM insulin. Cells on the coverslips were fixed in paraformaldehyde (3.5%) and mounted on to glass slides. The coverslips were examined for translocation of GFP-Glut 4 under laser scanning confocal microscope (Leica Corp., Rockleigh, NJ) (see **Fig 2b**).

### Extraction and isolation of lupinoside PA4 from Pueraria tuberosa:

*Pueraria tuberosa* root (1kg was cut finely and extracted with methanol (3x1.5L). The extracted solution, after evaporation in vacuo, gave a residue (90g) and was evaluated for bioactivity. The methanol extract was partitioned between ethyl acetate and water. The aqueous layer was further extracted with n- butanol. Removal of the solvent in vacuo from ethyl acetate-soluble portion, n-butanol-soluble portion and aqueous phase yielded 2.5g, 12g, and 64g of fraction respectively. Each fraction was tested for bioactivity and activity was found in n-butanol fraction. This fraction was subjected to Diaion HP-20 chromatography with water and methanol as eluent. The methanol eluent was evaporated to dryness (2.4g) and was further subjected to Sephadex LH-20 chromatography using methanol-water (1:1) and methanol as fluent. Evaporation of methanol-water fraction under reduced pressure yielded a solid (1.4g) that showed biological activity. Preparative

HPLC (µ-Bondapak, C-18 reverse phase column, methanol-1% aqueous acetic acid (7:3), flow 12mm/min and UV as detector as 210nm) of this solid furnished a homogeneous compound identified as lupinoside PA₄ (L PA₄)³² (0.28g) whose structure was determined by 1D, 2D NMR and Q-TOF-MS and some chemical reactions (see **Fig 3a**).

**Effect of LPA₄ treatment on palmitate induced inhibition of glucose uptake**. 3T3L1 adipocytes were treated for 24h in the absence and presence of LPA4 (20µg/ml) and palmitate (0.75mM) followed by 30min incubation with 100nM insulin in Kreb's Ringer Phosphate (KRP) buffer (12.5 mM HEPES, pH 7.4, 120 mM NaCl, 6 mM KCl, 1.2 mM MgSO₄, 1 mM CaCl₂, 0.4 mM NaH₂PO₄, 0.6 mM Na₂HPO₄) supplemented with 0.2 % bovine serum albumin and then ³H-2-DOG (0.4 nmoles) was added to each incubation 5 min prior to the termination of incubation. 3T3L1 cells were washed thrice with ice-cold KRP buffer in the presence of 0.3 mM phloretin to correct the glucose uptake data from simple diffusion and non specific trapping of radioactivity. Cells were solubilized with 1% NP-40 and [³H]-deoxyglucose was measured in a Liquid Scintillation counter (Packard, Tricarb 2100 TR) (see **Fig3b**)

3T3L1 cells incubated in an identical manner in the absence or presence of LPA₄ and palmitate were lysed by sonication and lysates were detected for p-IR and p-Akt as described above (see **Fig 3c**). In another set of experiment cells were transfected with GFP-Glut4 as described above followed by incubation without or with LPA₄ and palmitate for 24h. Cells were then incubated with 100nM insulin and Glut4 translocation was monitored under a confocal microscope (see **Fig 3d**).

### REFERENCES

1. Amos, A.F., McCarty, D.J., & Zimmet, P. The rising global burden of diabetes and its complications: estimates and projections to the year 2010. Diab Med. 14, S1-85 (1997).
2. Kopelman, P.G., Obesity as a Medical Problem. Nature 404, 635-643 (2000).
3. Klip, A. & Paquet, M.R. Glucose transport and glucose transporters in muscle and their metabolic regulation. Diabetes Care 13, 228-243 (1990).
4. DeFronzo, R.A. The triumvariate: beta cell, muscle, liver: a collusion responsible for NIDDM. Diabetes 37, 667-687 (1988).
5. Kelley, D.E., Mokan, M., Simoneau, J.A., & Mandarino, L.J. Interaction between glucose and free fatty acid metabolism in human skeletal muscle. J. Clin. Invest. 92, 91-98 (1993).
6. Boden, G., Chen, X., Ruiz, J., White, J.V., & Rossetti, L. Mechanisms of fatty acid-induced inhibition of glucose uptake. J. Clin. Invest. 93, 2438-2446 (1994).
7. Shulman, G.I. Cellular mechanisms of insulin resistance. J. Clin. Invest. 106, 171-176 (2000).
8. Dresner, A., Laurent, D., Marcucci, M., Griffin, M.E., Dufour, S., Cline, G.W., Slezak, L.A., Andersen, D.K., Hundal, R.S., Rothman, D.L., Petersen, K.F., & Shulman, G.I. Effects of free fatty acids on glucose transport and IRS-1-associated phosphatidylinositol 3-kinase activity. J. Clin. Invest.103, 253-259 (1999).
9. Pan, D.A., Lillioja, S., Kriketos, A.D., Milner, M.R., Baur, L.A., Bogardus, C., Jenkins, A.B. & Storlien, L.H. Skeletal muscle triglyceride levels are inversely related to insulin action. Diabetes 46, 983-988 (1997).
10. Boden, G.A. Role of fatty acids in the pathogenesis of insulin resistance and NIDDM. Diabetes 46, 3-10 (1997).
11. Itani, S.I., Ruderman, N.B., Frank, S., & Boden, G. Lipid induced insulin resistance in human muscle is associated with changes in diacylglycerol, Protein kinase C, and Ikb-α.. Diabetes 51, 2005-2011 (2002).
12. Chavez, J.A. Knotts, T.A., Wang Li-Ping, Li.G., Dobrowsky, R.T., Florant, G.L., & Summers, S.A. Role of ceramide but not diacylglycerol, in the Antagonism of Insulin Signal Transduction by Saturated Fatty Acids. J. Biol. Chem. 278, 10297-10303 (2003).
13. Griffin, M.E., Marcucci, M.J., Cline, G.W., Bell, K., Barucci, N., Lee, D., Goodyear, L.J., Kraegen, E.W., White, M. F., & Shulman, G.I. Free fatty acid-induced insulin resistance is associated with activation of protein kinase C theta and alterations in the insulin-signalling cascade. Diabetes 48, 1270-1274 (1999).
14. Beeson, M., Sajan, M.P., Dizon, M., Grebenev, D., Gomez-Daspet, J., Miura, A., Kanoh,Y., Powe, J., Bandyopadhyay, G., Standaert, M.L., & Farese, R.V. Activation of Protein Kinase C-zeta by Insulin and Phosphatidylinositol-3, 4,5-(PO (4))(3) Is Defective in Muscle in Type 2 Diabetes and Impaired Glucose Tolerance: Amelioration by Rosiglitazone and Exercise. Diabetes 52, 1926-34 (2003).
15. Yu, C., Chen, Y., Cline, G.W., Zhang, D., Zong, H., Wang, Y., Bergeron, R., Kim, J.K., Cushman, S.W., Cooney, G.J., Atcheson, B., White, M.F., Kraegen, E.W., & Shulman, G.I. Mechanisms by which fatty acids inhibit insulin activation of insulin receptor substrate-1 (IRS-1)-associated phosphatidylinositol 3-kinase activity in muscle. J. Biol. Chem. 27, 50230-50236 (2002).
16. Lehmann, J.M., Moore, L.B., Smith-Oliver, T.A., Wilkison, W.O., Willson, T.M., & Kliewer, S.A. An Antidiabetic Thiazolidinedione Is a High Affinity Ligand for Peroxisome Proliferator-activated Receptor γ (PPARγ). J. Biol. Chem, 270 12953 - 12956 (1995).
17. Martin, G., Schoonjans, K., Lefebvre, A.M., Staels, B. & Auwerx, J. Coordinate Regulation of the Expression of the Fatty Acid Transport Protein and Acyl-CoA Synthetase Genes by PPARα and PPARγ Activators. J. Biol. Chem, 272, 28210-28217 (1997).
18. Stumvoll, M., & Haring, H.U. Glitazones. Clinical effects and molecular mechanisms. Ann. Med. 34, 217-224 (2002).
19. Guan, H.P., Li, Y., Jensen, M.V., Newgard, C.B., Steppan, C.M.&. Lazar, M.A. A futile metabolic cycle activated in adipocytes by antidiabetic agents. Nat. med. 8, 1122 - 1128 (2002).
20. Reynoso, R., Salgado, L.M., & Calderon, V. High levels of palmitic acid lead to insulin resistance due to changes in the level of phosphorylation of the insulin receptor and insulin receptor substrate-1. Mol. Cell. Biochem, 246, 155-162 (2003).
21. Storz, P., Doppler, H., Wernig, A., Pfizenmaier, K., & Muller, G. Cross-talk mechanisms in the development of insulin resistance of skeletal muscle cells Palmitate rather than tumour necrosis factor inhibits insulin-dependent protein kinase B (PKB)/Akt stimulation and glucose uptake. Eur. J. Biochem. 266, 17-25 (1999).
22. Chavez, J.A., & Summers, S.A. Characterizing the effects of saturated fatty acids on insulin signaling and ceramide and diacylglycerol accumulation in 3T3-L1 adipocytes and C2C12 myotubes. Arch. Biochem. Biophys. 15, 101-9 (2003).
23. Weigert, C., Klopfer, K., Kausch, C., Brodbeck, K., Stumvoll, M., Hans, U. H, & Erwin D. S. Palmitate-Induced Activation of the Hexosamine Pathway in Human Myotubes Increased Expression of Glutamine:Fructose-6-Phosphate Aminotransferase. Diabetes 52, 650-656 (2003).
24. Salzman, A., Wan, C.F., & Rubin, C.S. Biogenesis, transit, and functional properties of the insulin proreceptor and modified insulin receptors in 3T3-L1 adipocytes. Use of monensin to probe proreceptor cleavage and generate altered receptor subunits. Biochemistry 23, 6555-65 (1984).
25. Velicelebi, G., & Aiyer, R.A. Identification of the alpha beta monomer of the adipocyte insulin receptor by insulin binding and autophosphorylation. Proc. Natl. Acad. Sci. U S A. 81, 7693-7 (1984).
26. Kohanski, R.A. & Lane, M.D. Homogeneous functional insulin receptor from 3T3-L1 adipocytes. Purification using N alpha Bl- (biotinyl-epsilon-aminocaproyl) insulin and avidin-sepharose. J. Biol. Chem., 260, 5014 - 5025 (1985).
27. Bélanger, C., Ansanay, H., Qanbar, R. & Bouvier, M. Primary sequence requirements for S-acylation of β-adrenergic receptor peptides. FEBS Letters, 499, 59-64 (2001).
28. Kennedy, M.E. & Limbird, L.E. Palmitoylation of the alpha 2A-adrenergic receptor. Analysis of the sequence requirements for and the dynamic properties of alpha 2A- adrenergic receptor palmitoylation. J. Biol. Chem., 269, 31915 - 31922 (1994).
29. Moffett, S., Rousseau, G., Lagacé, M. & Bouvier, M. The palmitoylation state of the β2-adrenergic receptor regulates the synergistic action of cyclic AMP-dependent protein kinase and β-adrenergic receptor kinase involved in its phosphorylation and desensitization. J. Neurochem. 76, 269-279 (2001).
30. Garrett, T.P.J., McKern, N.M., Lou, M.Z., Frenkel, M.J., Bentley, J.D., & Lovrecz, G.O. Crystal structure of the rst three domains of the type-1 insulin-like growth factor receptor. Nature 394, 395-399 (1998).
31. Adams, T.E., Epa, V.C., Garrett, T.P.J. & Ward, C.W. Structure and function of the type 1 insulin-like growth factor receptor. Cell. Mol. Life Sci. 57, 1050-1093 (2000).
32. Kinjo, J., Kishida, F., Watanable, K., Hashimoto, F. & Nohara, T. Five new triterpene glycosides from Russell lupine. Chem.Pharm.Bull. 42, 1874-1878 (1994).
33. Martin, S., Millar, C.A., Lyttle, C.T., Meerloo, T., Marsh, B.J., Gould, G.W., & James, D.E. Effects of insulin on intracellular GLUT4 vesicles in adipocytes: evidence for a secretory mode of regulation. J. Cell Sci. 113, 3427-3438 (2000).
34. Pessin, J.E., Thurmond, D.C., Elmendorf, J.S., Coker, K.J., & Okada, S. Molecular Basis of Insulin-stimulated GLUT4 Vesicle Trafficking. J. Biol. Chem, 274, 2593 - 2596 (1999).
35. Kupriyanova, T.A &. Kandror, K.V. Akt-2 Binds to Glut4-containing Vesicles and Phosphorylates Their Component Proteins in Response to Insulin. J. Biol. Chem., 274, 1458 -1464 (1999).
36. Gorski, J., Nawrocki, A., & Murthy, M. Characterization of free and glyceride-esterified long chain fatty acids in different skeletal muscle types of the rat. Mol. Cell. Biochem, 178, 113-118 (1998).
37. Vessby, B., Aro, A., Skarfors, E., Berglund, L., Salminen, I., & Lithell, H. The risk to develop NIDDM is related to the fatty acid composition of the serum cholesterol esters. Diabetes 43, 1353-1357 (1994).
38. Ho, R.C., Davy, K.P., Hickey, M.S., Summers, S.A., & Melby, C.L. Behavioral, metabolic, and molecular correlates of lower insulin sensitivity in Mexican-Americans. Am. J. Physiol. Endocrinol. Metab, 283, 799-808 (2002).
39. Turcotte, L.P., Swenberger, J.R., Zavitz Tucker, M., and Yee, A.J. Increased Fatty Acid Uptake and Altered Fatty Acid Metabolism in Insulin-Resistant Muscle of Obese Zucker Rats. Diabetes 50, 1389-1396, (2001).

## Claims

1. Use of a pharmaceutically effective amount of butanol fraction of extract of plant Pueraria tuberosa or Lupinoside PA4, optionally along with pharmaceutically available additive(s) for the manufacture of a medicament for preventing and/or treating diabetes type 2 in a subject in need thereof.

2. Use as claimed in claim 1, wherein the subject is an animal.

3. Use as claimed in claim 1, wherein the subject is a human being.

4. Use as claimed in claim 1, wherein the extract is obtained from root of the plant.

5. Use as claimed in claim 1, wherein the additive is selected from a group comprising nutrients such as proteins, carbohydrates, sugars, talc, magnesium stearate, cellulose, calcium carbonate, starch, gelatin paste, pharmaceutically acceptable carrier, excipients, diluent and, solvent.

6. Use as claimed in claim 1, wherein the fraction is administered at the concentration ranging between 1 to 40 mg /kg body weight.

7. Use as claimed in claim 1, wherein the Lupinoside is administered at the concentration ranging between 1 to 40 mg /kg body weight.

8. Use as claimed in claim 1, wherein the administration route is selected from a group comprising orally, intravenously, intramuscularly, and subcutaneously.

9. A pharmaceutical composition useful in preventing and/or treating diabetes type 2, said composition comprising of butanol fraction of extract of plant Pueraria tuberosa containing Lupinoside PA4 and additive(s).

10. A pharmaceutical composition as claimed in claim 9, wherein the additive is selected from a group comprising nutrients such as proteins, carbohydrates, sugars, talc, magnesium stearate, cellulose, calcium carbonate, starch, gelatin paste, pharmaceutically acceptable carrier, excipients, diluent and, solvent.

11. A pharmaceutical composition as claimed in claim 9, the extract is obtained from root of the plant.

12. A pharmaceutical composition as claimed in claim 9, the fraction is of concentration ranging between 1 to 40 mg/kg body weight.

13. A pharmaceutical composition as claimed in claim 9, wherein the composition is in a form selected from a group comprising capsule, syrup, concentrate, powder, and granules.

14. A pharmaceutical composition as claimed in claim 9, wherein the extract is an aqueous extract.

15. Use in accordance with any one of claims 1-14 wherein said active n-butanol fraction and active molecule Lupinoside PA (LPA4), useful in preventing and/or treating diabetes type 2, are obtained by a process comprising the steps of:
a. cutting the plant parts into small parts,
b. extracting the cut parts with methanol and water,
c. partitioning the methanol and water extract between ethyl acetate and water,
d. extracting the aqueous layer further with n-butanol to obtain butanol fraction, and
e. subjecting the n-butanol fraction to chromatography with water and methanol as eluent to obtain Lupinoside PA4 (LPA4).

16. Use as claimed in claim 15, wherein the plant part is root.

17. Use as claimed in claim 15, wherein the solvent is selected from a group comprising methanol, and water.

18. Use as claimed in claim 15, wherein the water and methanol are in the ratio of about

19. Use as claimed in claim 15, wherein the chromatography is column chromatography.

## Patentansprüche

1. Verwendung einer pharmazeutisch wirksamen Menge der Butanolfraktion eines Extraktes der Pflanze Pueraria tuberosa oder dem Lupinosid PA 4, wahlweise zusammen mit pharmazeutisch verträglichem Additiv(en) zur Herstellung eines Medikaments zur Vorbeugung und/oder Behandlung von Diabetes Typ 2 in einem Individuum, welches hierfür Bedarf hat.

2. Verwendung nach Anspruch 1, wobei das Individuum ein Tier ist.

3. Verwendung nach Anspruch 1, wobei das Individuum ein Mensch ist.

4. Verwendung nach Anspruch 1, wobei der Extrakt aus der Wurzel der Pflanze erhalten wird.

5. Verwendung nach Anspruch 1, wobei das Additiv ausgewählt wird aus der Gruppe, umfassend Nährmittel wie Proteine, Kohlenhydrate, Zucker, Talkum, Magnesiumstearat, Zellulose, Calciumkarbonat, Stärke, Gelatinepaste, pharmazeutisch verträgliche Träger, Excipienten, Verdünnungsmittel und Lösungsmittel.

6. Verwendung nach Anspruch 1, wobei die Fraktion in einer Konzentration von 1 bis 40 mg /kg Körpergewicht verabreicht wird.

7. Verwendung nach Anspruch 1, wobei das Lupinosid in einer Konzentration von 1 bis 40 mg /kg Körpergewicht verabreicht wird.

8. Verwendung nach Anspruch 1, wobei der Verabreichungsweg ausgewählt wird aus der Gruppe, umfassend eine orale, intravenöse, intramuskuläre und subkutane Verabreichung.

9. Pharmazeutische Zusammensetzung, die sich zur Vorbeugung und/oder Behandlung von Diabetes Typ 2 eignet, wobei die Zusammensetzung eine Butanolfraktion des Extrakts der Pflanze Pueraria tuberosa, die Lupinosid PA 4 enthält und Additiv(e) umfasst.

10. Pharmazeutische Zusammensetzung nach Anspruch 9, wobei das Additiv ausgewählt wird aus der Gruppe, bestehend aus der Gruppe, umfassend Nährmittel wie Proteine, Kohlenhydrate, Zucker, Talkum, Magnesiumstearat, Zellulose, Calciumkarbonat, Stärke, Gelatinepaste, pharmazeutisch verträgliche Träger, Excipienten, Verdünnungsmittel und Lösungsmittel.

11. Pharmazeutische Zusammensetzung nach Anspruch 9, wobei der Extrakt aus der Wurzel der Pflanze erhalten wird.

12. Pharmazeutische Zusammensetzung nach Anspruch 9, wobei die Fraktion eine Konzentration von 1 bis 40 mg/ kg Körpergewicht besitzt.

13. Pharmazeutische Zusammensetzung nach Anspruch 9, wobei die Zusammensetzung in einer Form vorliegt, ausgewählt aus der Gruppe, bestehend aus einer Kapsel, Sirup, Konzentrat, Pulver und Körnchen.

14. Pharmazeutische Zusammensetzung nach Anspruch 9, wobei der Extrakt ein wässriger Extrakt ist.

15. Verwendung nach einem der Ansprüche 1 bis 14, wobei die Aktive n-Butanol-Fraktion und das aktive Lupinosid PA (LPA 4)-Molekül, welches sich zur Vorbeugung und/oder Behandlung von Diabetes Typ 2 eignet, durch ein Verfahren erhalten werden, welches die nachfolgenden Schritte umfasst:
a. Schneiden der Pflanzenteile in kleine Teile,
b. Extrahieren der geschnittenen Teile mit Methanol und Wasser,
c. Aufteilen des Methanol- und Wasserextraktes zwischen Ethylacetat und Wasser,
d. Weiteres Extrahieren der wässrigen Schicht mit n-Butanol, um eine Butanolfraktion zu erhalten, und
e. Chromatographie der n-Butanol-Fraktion mit Wasser und Methanol als Elutionsmittel, um Lupinosid PA 4 (LPA 4) zu erhalten.

16. Verwendung nach Anspruch 15, wobei der Pflanzenteil eine Wurzel ist.

17. Verwendung nach Anspruch 15, wobei das Lösungsmittel ausgewählt wird aus der Gruppe, umfassend Methanol und Wasser.

18. Verwendung nach Anspruch 15, wobei das Wasser und Methanol im Verhältnis von etwa 1:1 vorliegen.

19. Verwendung nach Anspruch 15, wobei die Chromatographie eine Säulenchromatographie ist.

## Revendications

1. Utilisation d'une quantité efficace d'un point de vue pharmacologique d'une fraction d'alcool butylique d'extrait de plante Pueraria tuberosa ou Lupinoside A P4, de manière facultative en même temps qu'un ou des additif(s) disponibles d'un point de vue pharmacologique pour la fabrication d'un médicament pour empêcher et/ou traiter le diabète de type 2 chez un sujet en ayant besoin.

2. Utilisation selon la revendication 1, dans laquelle le sujet est un animal.

3. Utilisation selon la revendication 1, dans laquelle le sujet est un être humain.

4. Utilisation selon la revendication 1, dans laquelle l'extrait est obtenu à partir de la racine de la plante.

5. Utilisation selon la revendication 1, dans laquelle l'additif est sélectionné à partir d'un groupe comprenant des substances nutritives comme des protéines, des hydrates de carbone, des sucres, le talc, le stéarate de magnésium, la cellulose, le carbonate de calcium, l'amidon, la pâte de gélatine, un vecteur acceptable d'un point de vue pharmacologique, des excipients, un diluant et un solvant.

6. Utilisation selon la revendication 1, dans laquelle la fraction est administrée à une concentration comprise entre 1 et 40 mg / kg de poids corporel.

7. Utilisation selon la revendication 1, dans laquelle le Lupinoside est administré à une concentration comprise entre 1 et 40 mg / kg de poids corporel.

8. Utilisation selon la revendication 1, dans laquelle la voie d'administration est sélectionnée à partir d'un groupe comprenant les voies orale, intraveineuse, intramusculaire et sous-cutanée.

9. Composition pharmaceutique utile dans la prévention et/ou le traitement du diabète de type 2, ladite composition comprenant une fraction d'alcool butylique d'extrait de plante Pueraria tuberosa contenant un Lupinoside A P4 et un ou des additif(s).

10. Composition pharmaceutique selon la revendication 9, dans laquelle l'additif est sélectionné à partir d'un groupe comprenant des substances nutritives comme des protéines, des hydrates de carbone, des sucres, le talc, le stéarate de magnésium, la cellulose, le carbonate de calcium, l'amidon, la pâte de gélatine, un vecteur acceptable d'un point de vue pharmacologique, des excipients, un diluant et un solvant.

11. Composition pharmaceutique selon la revendication 9, dans laquelle l'extrait est obtenu à partir de la racine de la plante.

12. Composition pharmaceutique selon la revendication 9, dans laquelle la fraction a une concentration comprise entre 1 et 40 mg / kg de poids corporel.

13. Composition pharmaceutique selon la revendication 9, dans laquelle la composition est sous une forme sélectionnée à partir d'un groupe comprenant la capsule, le sirop, le concentré, la poudre et les granules.

14. Composition pharmaceutique selon la revendication 9, dans laquelle l'extrait est un extrait aqueux.

15. Utilisation selon l'une quelconque des revendications 1 à 14, dans laquelle ladite fraction active d'alcool n-butylique et la molécule active de Lupinoside A P (LA P4), utiles dans la prévention et/ou le traitement du diabète de type 2, sont obtenues par un processus comprenant les étapes consistant à :
a. couper les éclats de plante en petits morceaux,
b. extraire les morceaux coupés avec du méthanol et de l'eau,
c. séparer le méthanol et l'extrait aqueux entre de l'acétate d'éthyle et de l'eau,
d. extraire la couche aqueuse en outre avec de l'alcool n-butylique pour obtenir la fraction d'alcool butylique, et
e. soumettre la fraction d'alcool n-butylique à une chromatographie avec de l'eau et du méthanol en tant qu'éluant pour obtenir un Lupinoside A P4 (LA P4).

16. Utilisation selon la revendication 15, dans laquelle l'éclat de plante est la racine.

17. Utilisation selon la revendication 15, dans laquelle le solvant est sélectionné à partir d'un groupe comprenant le méthanol et l'eau.

18. Utilisation selon la revendication 15, dans laquelle l'eau et le méthanol sont dans un rapport d'environ 1 / 1.

19. Utilisation selon la revendication 15, dans laquelle la chromatographie est une chromatographie sur colonne.
